# EUROPEAN PATENT SPECIFICATION

(11) **EP 3 893 969 B1**
(45) Date of publication and mention of the grant of the patent: **28.05.2025**
(21) Application number: 19802210.5
(22) Date of filing: 20.11.2019
(51) Int. Cl.: A61M 5/32, A61M 5/20

(54) **ACTIVATION PREVENTION CAP ASSEMBLY**
AKTIVIERUNGSVERHINDERNDE KAPPENANORDNUNG
ENSEMBLE DE CAPUCHON DE PRÉVENTION D'ACTIVATION

(30) Priority: 12.12.2018 EP 18212091
(43) Date of publication of application: 20.10.2021
(73) Proprietor: SHL Medical AG, 6302 Zug (CH)
(72) Inventor: ALEXANDERSSON, Oscar, 6302 Zug (CH)
(86) International application number: PCT/EP2019/081897
(87) International publication number: WO 2020/120084

(56) References cited:
- EP-A1- 2 923 716
- WO-A1-2017/029032
- WO-A1-2017/207224

## Description

### TECHNICAL AREA

The present disclosure relates to an injection activation prevention system of a medicament delivery device and in particular to a cap assembly of a medicament delivery device that has an injection activation prevention feature.

### BACKGROUND OF INVENTION

Today's medicament delivery devices may be complex and involves many different components. The physical features of each component and the way components are assembled together has a direct effect on how these components are going to interact with each other and on the success rate of the medicament delivery device in use.

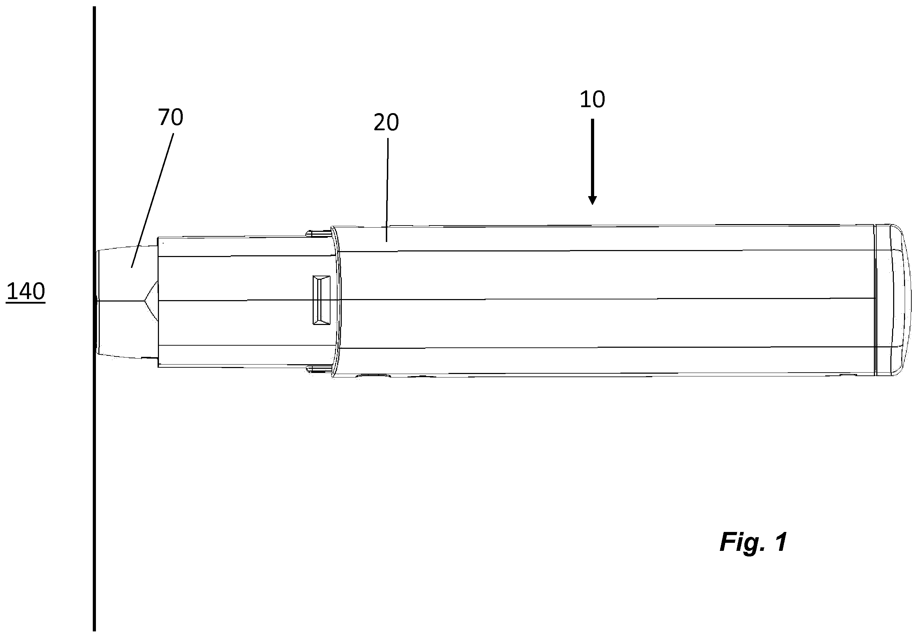

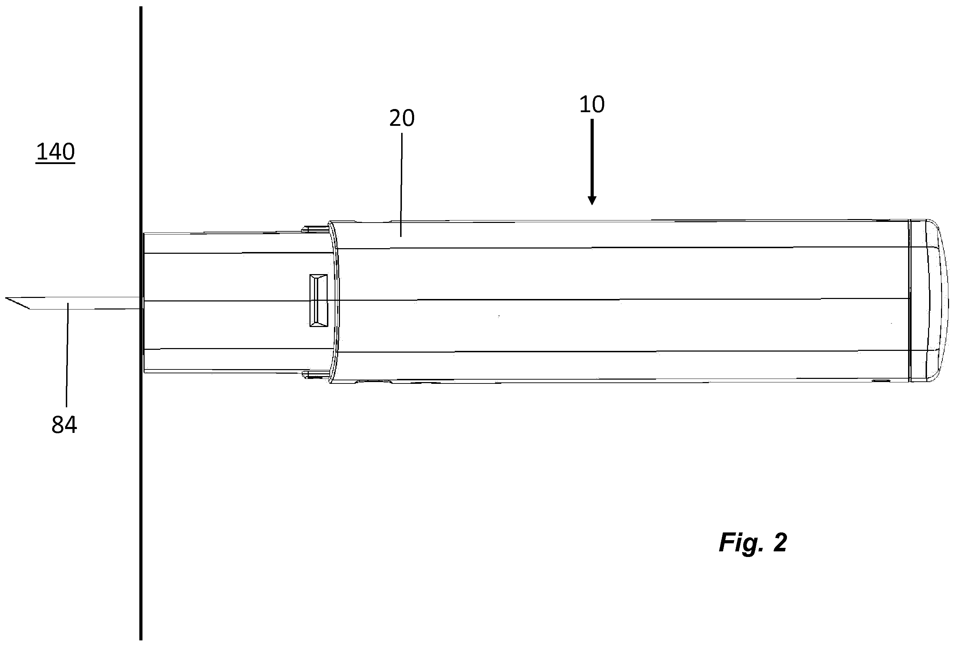

One such area is the use of a needle cover configured to be movable to activate the power pack within of a medicament delivery device for medicament injection. Fig. 1 is a front view of an example medicament delivery device just before dose delivery and Fig. 2 is a front view of the medicament delivery device during dose delivery. To initiate the injection process, the user places the medicament delivery device 10 on an injection site, such as injection site 140 as shown in Figure 1. When the drug delivery device 10 is pressed onto the injection site 140 in proximal direction, the needle cover 70 moves in a distal direction relative to the main housing 20. This retraction of the needle cover 70 exposes the needle 84 of a syringe (not illustrated) within the medicament delivery device 10 and the needle 84 is consequently inserted into the injection site 140. In addition to exposing the needle 84, this retraction of the needle cover 70 also serves to activate a power pack (not illustrated) within the medicament delivery device 10, wherein the power pack is configured to move a stopper of the syringe for medicament ejection through the needle 84 of the syringe and into the injection site 140.

However, during transportation the medicament delivery device 10 may be moved by externals various forces from sources such as the movement of the transportation vehicle, waves of the sea, or wind. Such forces may result in an unintended retraction of the needle cover 70 in the proximal direction relative to the main housing 20 which results in an accidental activation of the power pack and medicament ejection through the needle of the syringe. Thus, there is a need for ensuring that the needle cover is not moved to retract in the proximal direction by any force other than that applied by the user. Some other examples can be found in EP2932716A1, WO2017207224A1, and WO2017029032A1.

### BRIEF DESCRIPTION OF INVENTION

In the present disclosure, when the term "distal" is used, this refers to the direction pointing away from the dose delivery site. When the term "distal part/end" is used, this refers to the part/end of the delivery device, or the parts/ends of the members thereof, which under use of the medicament delivery device is/are located furthest away from the dose delivery site. Correspondingly, when the term "proximal" is used, this refers to the direction pointing to the dose delivery site. When the term "proximal part/end" is used, this refers to the part/end of the delivery device, or the parts/ends of the members thereof, which under use of the medicament delivery device is/are located closest to the dose delivery site.

Further, the term "longitudinal", with or without "axis", refers to a direction or an axis through the device or components thereof in the direction of the longest extension of the device or the component.

The term "lateral", with or without "axis", refers to a direction or an axis through the device or components thereof in the direction of the broadest extension of the device or the component. "Lateral" may also refer to a position to the side of a "longitudinally" elongated body.

In a similar manner, the terms "radial" or "transversal", with or without "axis", refers to a direction or an axis through the device or components thereof in a direction generally perpendicular to the longitudinal direction, e.g. "radially outward" would refer to a direction pointing away from the longitudinal axis.

Also, if nothing else is stated, in the following description wherein the mechanical structure of the device and the mechanical interconnection of its components is described, the device is in an initial non-activated or non-operated state.

In view of the foregoing, a general object of the present disclosure is to provide a needle shield remover for a cap of a medicament delivery device, which needle shield remover is easier to assemble.

These and other aspects of, and advantages with, the present disclosure will become apparent from the following detailed description of the invention and from the accompanying drawings.

The invention lies in the medicament delivery device of claim 1. Further embodiments are given in the dependent claims. One aspect of the disclosure is directed to

According to a main aspect of the disclosure it is characterized by a cap assembly for a medicament delivery device, wherein the medicament delivery device has a housing and a needle cover movable in relation to the housing. The cap assembly comprises a locking member including a flexible first engagement member configured to interact with the needle cover and the housing to limit a movement of the needle cover toward an inside of the housing and a cap configured to engage the locking member and movably arranged in relation to the housing and the locking member between a first position in which the cap is engaged to the housing and a second position in which the cap disengages the housing. One end of the housing is configured to engage the first engagement member to prevent the first engagement member from disengaging the needle cover when the cap is located between the first and second positions. The cap is further movably arranged between the second position and a third position in which the cap disengages the housing and in which the locking member disengages the needle cover and no longer limits the movement of the needle cover. Further, the needle cover includes a second engagement member configured to engage the first engagement member when the cap is between the first position and the second position, wherein the first engagement member disengages the second engagement member when the cap is moved from the second position to the third position.

The cap assembly also comprises a needle shield remover configured to engage the locking member and a needle shield attached to a medicament container within the needle cover and the housing, when the cap is moved from the second position to the third position the cap moves the locking member to move the needle shield remover away from the medicament container to pull the needle shield away from the medicament container.

The cap includes a third engagement member and the locking member includes a fourth engagement member, the third engagement member is configured to engage the fourth engagement member when the cap moves from the first position to the second position, the third engagement member moves the locking member away from the housing when the cap moves from the second position to the third position.

The cap includes a plurality of gripping protrusions on an outer surface of the cap configured to interact with a user when the user grips the cap.

Another aspect of the disclosure is directed to a medicament delivery device comprising a housing, a needle cover movably placed at least partially in the housing, a medicament container disposed within the needle cover and the housing, and the cap assembly as described above.

In one embodiment, the housing includes a fifth engagement member on an outer surface of the housing, the cap includes at least a sixth engagement member on an inner surface of the cap, the sixth engagement member is configured to engage the fifth engagement member in order for the cap to be mounted to the housing.

### BRIEF DESCRIPTION OF DRAWINGS

In the following detailed description of the invention, reference will be made to the accompanying drawings, of which
- Fig. 1: a front view of an example medicament delivery device just before dose delivery.
- Fig. 2: a front view of an example medicament delivery device during before dose delivery.
- Fig. 3: a front view of an example medicament delivery device during before the removal of cap assembly.
- Fig. 4: an exploded view of an example medicament delivery device of the first embodiment.
- Fig. 5: a perspective view of the proximal portion of the medicament delivery device according to the first embodiment.
- Fig. 6: a cross-section view of the medicament delivery device according to the first embodiment and before the removal of the cap assembly.
- Fig. 7: a cross-section view of the medicament delivery device according to the first embodiment and before the removal of the cap assembly.
- Fig. 8: a cross-section view of the medicament delivery device according to the first embodiment and after the removal of the cap assembly.
- Fig. 9: a perspective view of the medicament delivery device according to the first embodiment and pressed against an injection site.
- Fig. 10: a perspective view of the medicament delivery device according to the first embodiment after the power pack is activated.

### DETAILED DESCRIPTION OF THE INVENTION

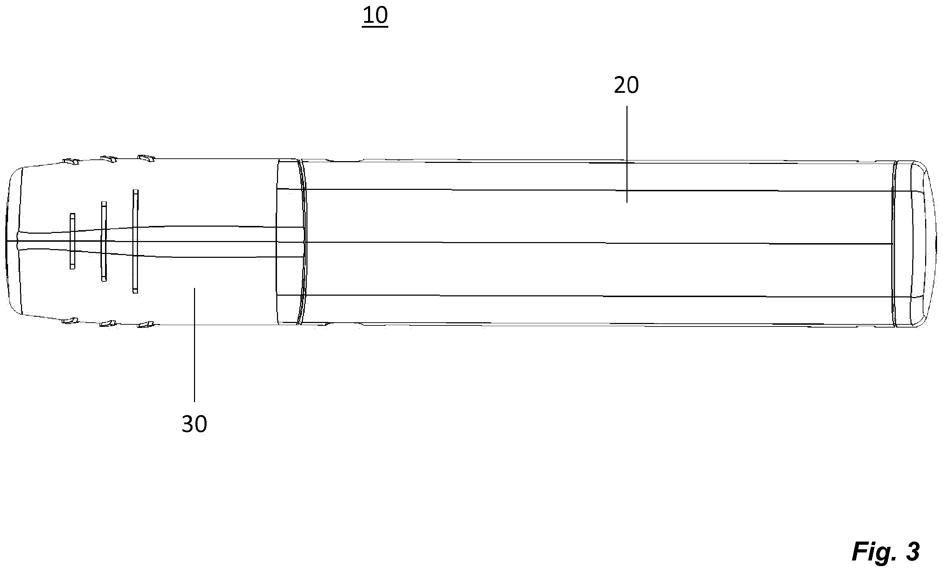

Fig. 3 shows a medicament delivery device 10 that includes a housing 20 and a cap assembly 30 coupling with the proximal end of the housing 20. The medicament delivery device 10 further includes a medicament container (not illustrated) disposed within the housing 20. The medicament container within the housing 20 contains medicament and a needle for injection site penetration and medicament injection that will be further explained in the later sections. The housing 20 has an opening at its proximal end that allows the needle of the medicament container to protrude outside the housing 20.

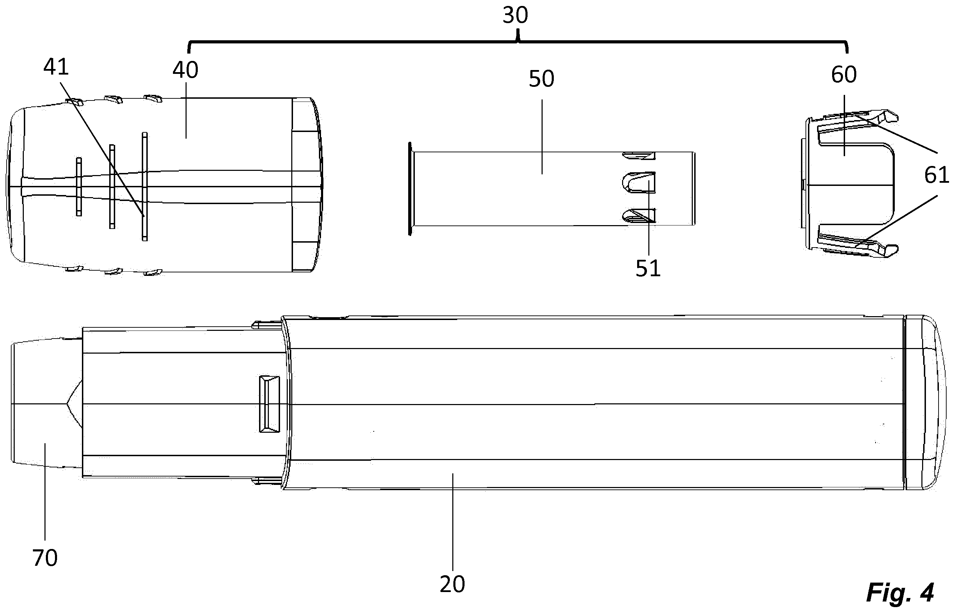

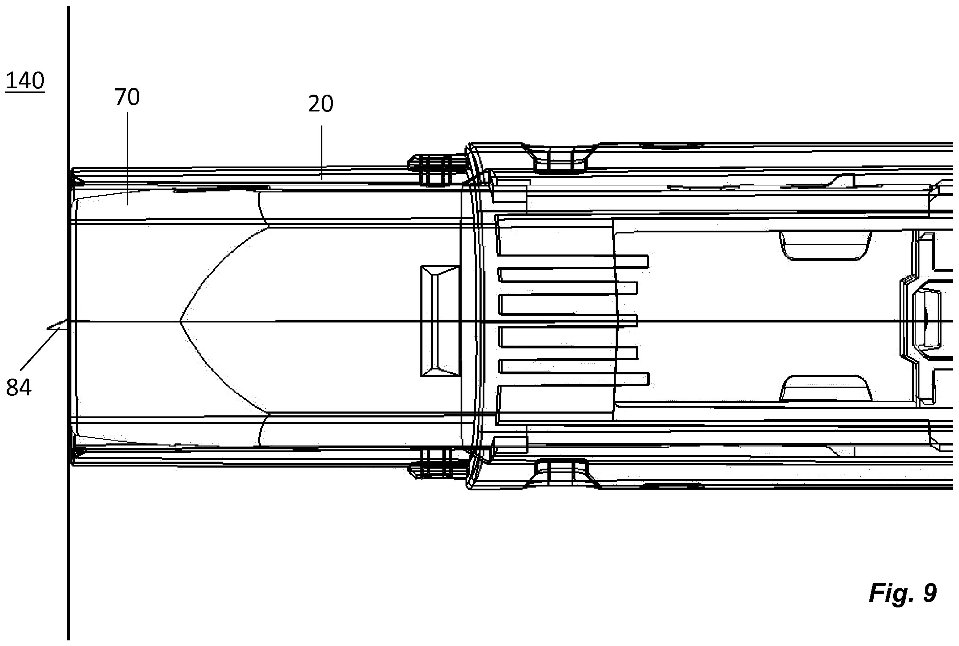

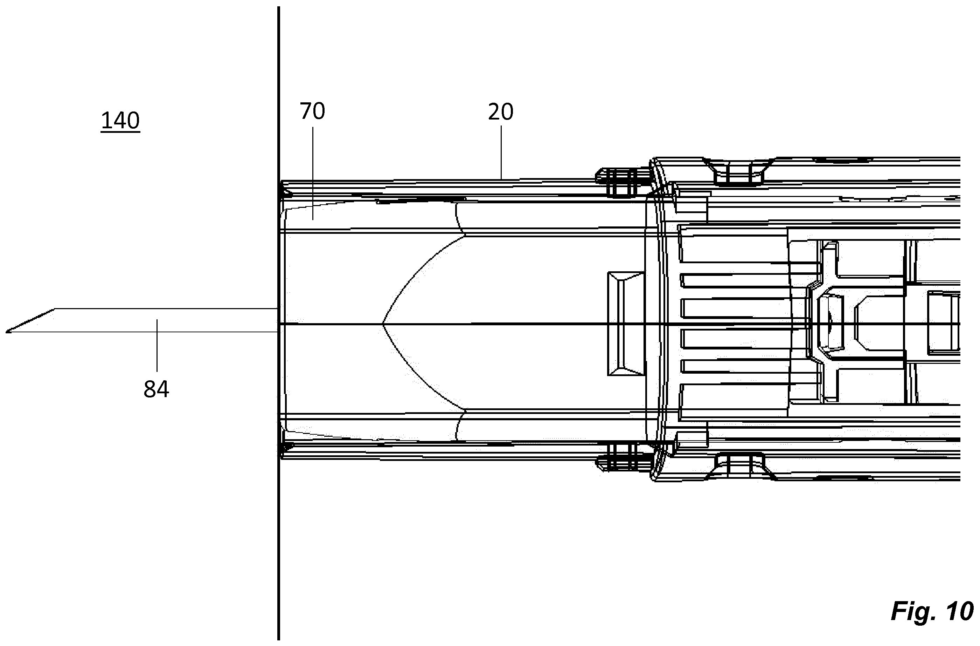

Fig. 4 shows an exploded view of the housing 20 and the cap assembly 30. In the present embodiment, the medicament delivery device 10 further includes a needle cover 70 movably arranged at least partially within the housing 20. After the removal of the cap assembly 30, the user can then place the needle cover 70 on an injection site to initiate the injection process. When the needle cover 70 is pressed onto the injection site 140 in distal direction same as illustrated in Fig. 2, the needle cover 70 moves in a distal direction relative to the housing 20. This retraction of the needle cover 70 exposes the needle 84 of a medicament container (illustrated in Figs. 9 and 10) within the medicament delivery device 10 and the needle 84 is consequently inserted into the injection site 140. In addition to exposing the needle 84, this retraction of the needle cover 70 also serves to activate a power pack (not illustrated) within the medicament delivery device 10, wherein the power pack is configured to move a stopper of the medicament container for medicament ejection through the needle 84 of the medicament container and into the injection site 140.

As illustrated in Fig. 4, the cap assembly 30 includes a cap 40, a needle shield remover 50, and a locking member 60. The cap 40 includes first engagement members that in the embodiment shown are a plurality of gripping protrusions 41 disposed on the outer surface of the cap 40, wherein the gripping protrusions 41 are configured to increase the grip of the user when holding and pulling the cap 40 with his/her fingers to remove the cap assembly 30 from the housing 20.

The needle shield remover 50 is configured to be coupled with the cap 30 so that later the user can pull both the cap 30 and needle shield remover 50 away from housing 20. The needle shield remover 50 is also configured to engage a needle shield attached to the medicament container to cover and protect the needle of the medicament container. More specifically, the needle shield remover 50 includes a plurality of prongs 51 configured to engage the outer surface of the needle shield so that when the cap 40 is pulled by the user the associated needle shield remover 50 can also pull the needle shield off the medicament container.

The locking member 60 is configured to engage the needle cover 70 when the whole cap assembly 30 is coupled with the housing 20 to cover both the proximal portion of the housing 20 and the needle cover 70. More specifically, the locking member 60 includes at least one engagement member 61 configured to engage the needle cover 70 to limit the movement of the needle cover 70 toward the inside of the housing 20 and prevent accidental activation of the power pack when the cap assembly 30 is still attached to the housing 20. As described above, the needle cover 70 retracted into the housing 20 will activate the power pack for the subsequent medicament injection. Thus, by preventing the needle cover 70 from accidental retraction, the locking member 60 and its engagement member 61 also prevent the accident activation of the power pack before the cap assembly 30 is removed from the housing 20. The interaction between the cap assembly 30 and needle cover 70 will be further explained in the later sections.

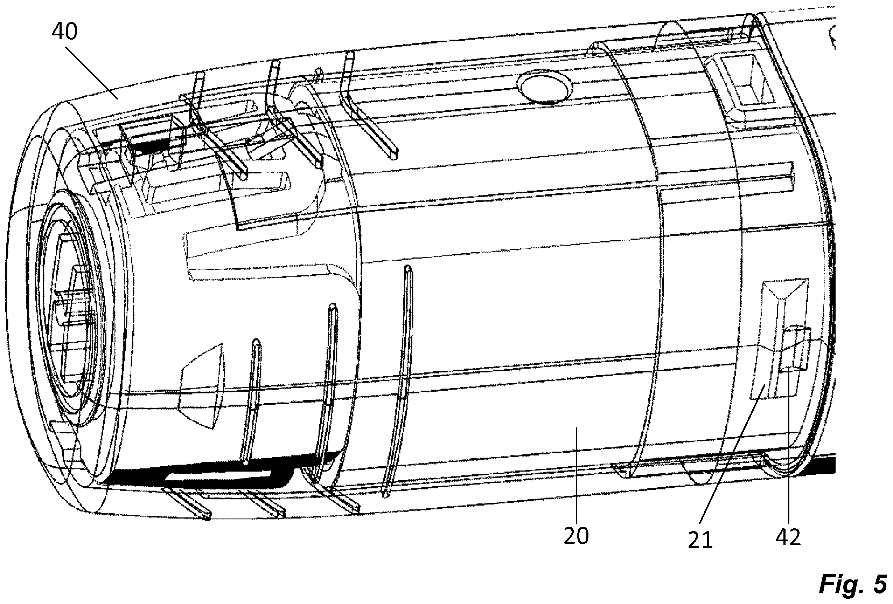

Fig. 5 is a perspective view of the cap assembly 30 coupled with the housing 20, wherein the cap 40 is made partially transparent to facilitate illustration. In the present embodiment, the cap 40 includes a housing engagement member 42 disposed on the inner surface of the cap 40. As illustrated, the housing engagement member 42 is a protrusion protruding radially inward from the inner surface of the cap 40. On the other hand, the housing 20 includes a cap engagement member 21 disposed on the proximal portion of the housing 20. The cap engagement member 21 is a protrusion protruding radially outward from the outer surface of the housing 20. In the present embodiment, to couple the cap 40 with the housing 20, the cap 40 must be pushed toward the distal end of the housing 20. During the process, the distal portion of the cap 40 flexes radially outward in order for the housing engagement member 42 to travel from the bottom on one side of the cap engagement member 21 to the top of said member 21. Then, the housing engagement member 42 travels from the top of the cap engagement member 21 to the bottom on the other side of said member 21. This completes the process of coupling the housing 20 and cap 40 during assembly. Afterwards, decoupling the cap 40 from the housing 20 requires not just the force to pull the cap 40 away from the housing 20, but also the additional force to flex the distal portion of cap 40 in order for the housing engagement portion 42 to travel over the cap engagement portion 21. This coupling feature prevents the cap 40 from disengaging the housing 20 during transportation due to random forces so that the cap 40 can continue to protect the proximal portion of housing 40 and needle cover 70 until it's actively removed by the user.

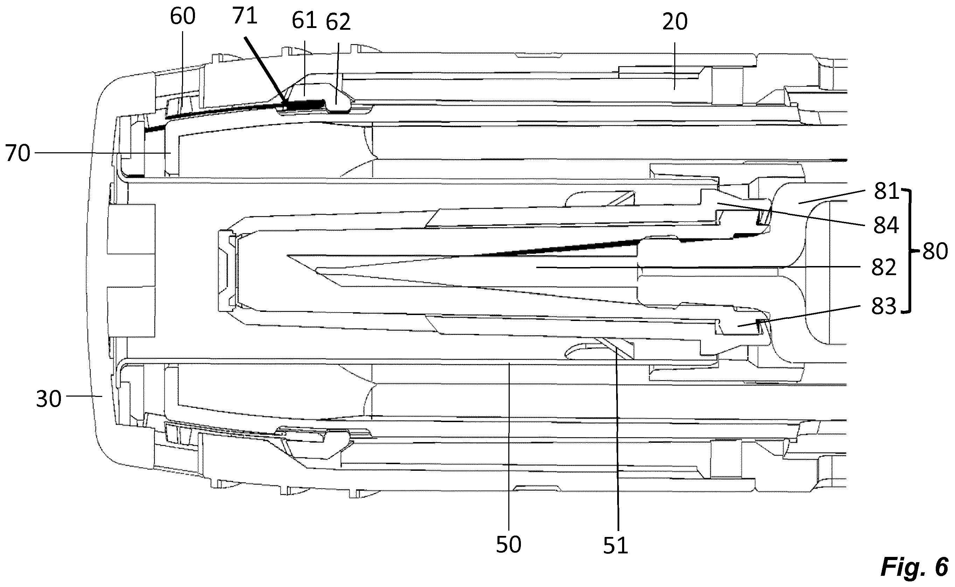

Fig. 6 shows a cross-section view of the medicament delivery device 10 according to the first embodiment and before the removal of the cap assembly 30. As illustrated, the medicament delivery device 10 includes a syringe 80 disposed in the housing 20. The syringe 80 includes a medicament container portion 81 and a needle 82 attached on the proximal portion of the medicament container portion 81. The syringe 80 further includes a flexible needle shield (FNS) 83 covering and protecting the needle 82 and a rigid needle shield (RNS) 84 covering and protecting the FNS 83. The needle cover 70 is disposed between the housing 20 and the syringe 80 while covering the needle 82. On the other hand, the needle shield remover 50 is disposed between the needle cover 70 and the RNS 84. In the present embodiment, the needle shield remover 50 is attached to the cap 30 and a locking member 60. The needle shield remover 50 includes inwardly-projecting gripping members 51 which engage the RNS 84, either by a distal end thereof or by engagement to a circumferential surface of the RNS 84, such that removal of the cap 30 pulls the needle shield remover 50 as well as the FNS 83 and the RNS 84 in the proximal direction off the medicament container portion 81 and away from the needle 82.

In the present embodiment, the locking member 60 is disposed between the cap 30 and needle cover 70. The needle cover 70 includes a groove 71 formed on its outer surface. On the other hand, the locking member 60 includes the engagement member 61 extending in a substantially distal direction and is configured to interact with the groove 71. The locking member 60 further includes a stop cam 62 disposed at the end of the engagement member 61 and resting within the groove 71. As described above, the needle cover 70 can be moved in a distal direction back into the housing 20 to activate the power pack (not illustrated) which then drives the syringe 80 in a proximal direction and the medicament out of the syringe 80 through the needle 82. Because the stop cam 62 is located within the groove 71, the needle cover 70 moving in the distal direction will collide with the stop cam 62 and is then prevented from moving further in the distal direction.

However, there is a possibility that excessive force may force the needle cover 70 to bias the engagement member 61 radially outward and then activate the power pack. To prevent such scenario, the end of the engagement member 61 is fitted into the space between the needle cover 70 and the proximal end of the housing 20. In this way, the proximal end of the housing 20 will absorb the force applied on stop arm 61 and thus prevent the engagement member 61 from flexing radially outward. In the present embodiment, the end of the engagement member 61 and the proximal end of the housing 20 have corresponding inclined surface. This allows the end of the engagement member 61 to be fitted into the space between the needle cover 70 and the proximal end of the housing 20.

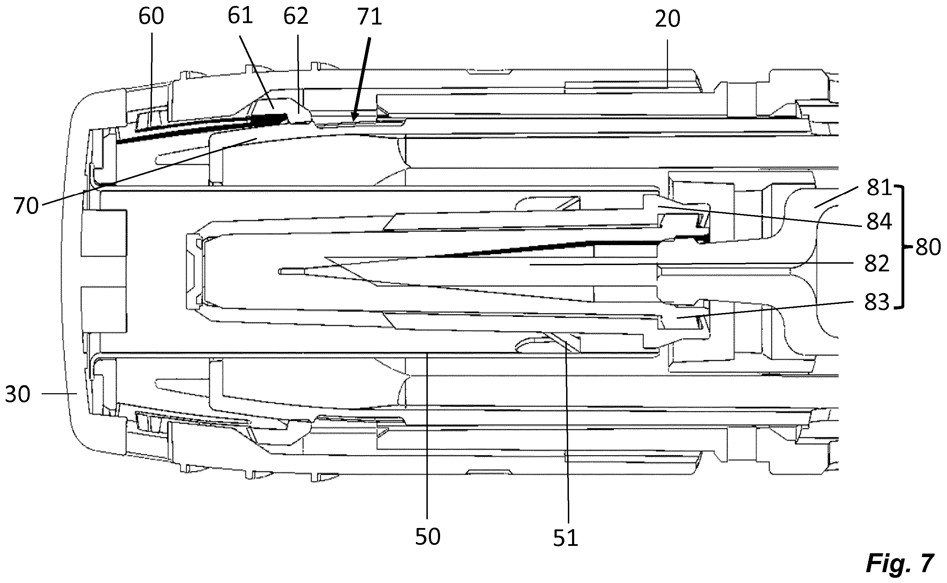

Fig. 7 is another cross-section view of the medicament delivery device according to the first embodiment and before the complete removal of the cap assembly. In Fig. 7, the cap assembly 30 is pulled slightly away from the housing 20. At this moment, the housing engagement member 42 of the cap 40 has travelled over the cap engagement member 21 of the housing 20 that allows the cap 40 to disengage from the housing 20.

Also, the stop cam 62 is no longer located within the groove 71 on the needle cover 70. As described above, the locking member 60 is coupled with both the cap 40 and the needle shield remover 50 to form the cap assembly 30. This allows the locking member 60 to move together with the cap 40 when pulled by the user. As the cap 40 is pulled by the user away from the housing 20, the locking member 60 and its stop cam 62 travels along the outer surface of the needle cover 70 and then leaves the groove 71. At this moment, there is nothing to stops the needle cover 70 from moving in a distal direction into the housing 20 to activate the power pack within. Thus, the user should only remove the cap assembly 30 when he/she is ready to use the medicament delivery device 10 for medicament injection. However, if the user changes mind and decides not the initiate the needle penetration and subsequent injector, the user can still couple the cap assembly 30 with the housing 20 to place the stop cam 62 back into the groove 71.

As mentioned above, the needle shield remover 50 is coupled with both the cap 40 and the locking member 60. Thus, when the cap 40 is pulled away from the housing 20, the gripping members 51 of the needle shield remover 50 engages the RNS 84 which engages the FNS 83. Thus, pulling the cap 40 will result in the removal of the FNS 83 and the RNS 84 away from the syringe 80 to expose the needle 82 on the syringe 80.

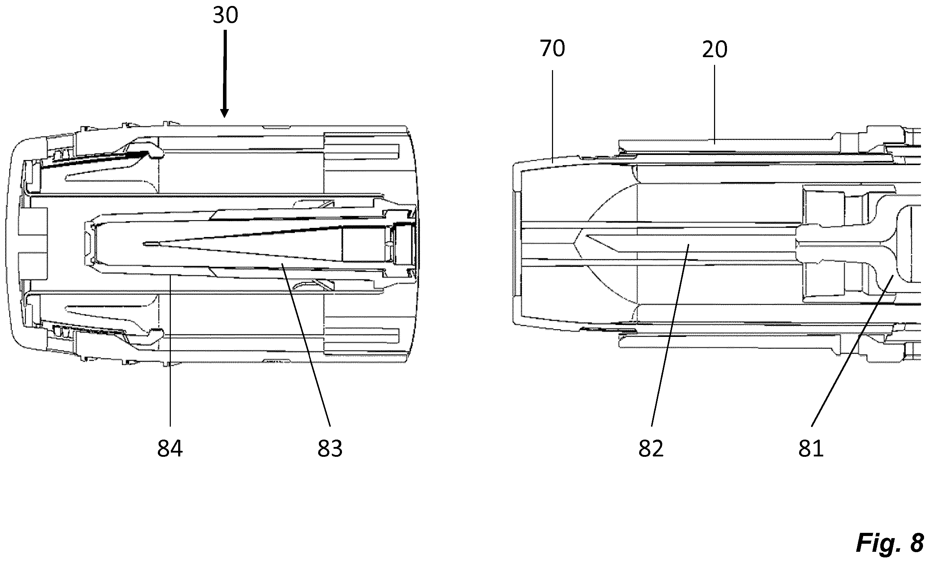

Fig. 8 is yet another perspective view of the medicament delivery device according to the first embodiment. As illustrated, the cap assembly 30 is removed from the housing 20 to expose the proximal portion of the housing 20 and the needle cover 70. Now the user can press the needle cover 70 on an injection site 140 for the subsequent needle penetration and medicament injection illustrated in Figs. 9 and 10.

Fig. 9 illustrates a perspective view of the medicament delivery device 10 with its needle cover 70 pressed against the injection site 140. At this moment, the needle cover 70 is retracted into the housing 20 and about to activate the power pack in the housing 20. Also, it can be seen that the needle 84 has been exposed outside the proximal end of the medicament delivery device 10 and penetrates the injection site 140 with its tip. Fig. 10 illustrates a perspective view of the medicament delivery device 10 with its power pack activated by the retracted needle cover 70. In Fig. 10, the distal end of the retracted needle cover 70 interacts with and activate the power pack which then biases the syringe 80 toward the injection site 140 for a deeper needle penetration. After the syringe 80 has reached its further position, the power pack then applies forces on a stopper within the syringe 80 axially movable within the housing 20 to bias the stopper to push the medicament in the syringe 80 out through the needle 82 and enter the injection site 140.

In the Figures, various engagement features for are shown for providing an engagement between one or more components of the drug delivery device. The engagement features may be any suitable connecting mechanism such as a snap lock, a snap fit, form fit, a bayonet, lure lock, threads or combination of these designs. Other designs are possible as well.

It should be understood that the illustrated components are intended as an example only. In other example embodiments, fewer components, additional components, and/or alternative components are possible as well. Further, it should be understood that the above described and shown embodiments of the present disclosure are to be regarded as non-limiting examples and that they can be modified within the scope of the claims.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims, along with the full scope of equivalents to which such claims are entitled.

## Claims

1. A medicament delivery device (10) comprising:
a housing (20);
a needle cover (70) movably placed at least partially in the housing;
wherein the medicament delivery device further comprises a medicament container disposed within the needle cover and the housing; and
a cap assembly (30) configured to be mounted to the housing to cover a proximal opening of the housing, wherein the cap assembly comprises
a locking member (60) including a first engagement member (61) configured to interact with the needle cover and the housing to limit a movement of the needle cover toward an inside of the housing, wherein the first engagement member is radially flexible and disposed between the housing and the needle cover; and
a cap (40) configured to engage the locking member and movably arranged in relation to the housing and the locking member between a first position in which the cap is engaged to the housing and a second position in which the cap disengages the housing;
wherein the needle cover includes a second engagement member (71) configured to engage the first engagement member when the cap is between the first position and
the second position;
wherein the second engagement member is a groove formed on the outer surface of the needle cover, and wherein the locking member further includes a stop cam (62) disposed at the end of the first engagement member configured to rest within the groove of the needle cover;
wherein the cap is further movably arranged between the second position and a third position in which the stop cam of the locking member disengages the groove of the needle cover and no longer limits the movement of the needle cover
wherein the cap includes a third engagement member and the locking member includes a fourth engagement member, the third engagement member is configured to engage the fourth engagement member when the cap moves from the first position to the second position, and the third engagement member is configured to move the locking member away from the housing when the cap moves from the second position to the third position.

2. The medicament delivery device of claim 1, **characterized in that** an end of the housing is configured to engage the first engagement member to prevent the first engagement member from moving away from the needle cover when the cap is located between the first and second positions.

3. The medicament delivery device of claim 1, **characterized in that** the cap assembly further comprising a needle shield remover (50) configured to engage the locking member and a needle shield (84) attached to a medicament container within the needle cover and the housing, when the cap is moved from the second position to the third position the cap moves the locking member to move the needle shield remover away from the medicament container to pull the needle shield away from the medicament container.

4. The medicament delivery device of claim 1, **characterized in that** the cap includes a plurality of gripping protrusions (41) on an outer surface of the cap configured to interact with a user when the user grips the cap.

5. The medicament delivery device of claim 1, **characterized in that** the housing includes a fifth engagement member (21) on an outer surface of the housing, the cap includes at least a sixth engagement member (42) on an inner surface of the cap, the sixth engagement member is configured to engage the fifth engagement member in order for the cap to be mounted to the housing.

## Patentansprüche

1. Arzneimittelabgabevorrichtung (10), umfassend:
ein Gehäuse (20);
eine Nadelabdeckung (70), die mindestens teilweise in dem Gehäuse bewegbar platziert ist;
wobei die Arzneimittelabgabevorrichtung ferner einen Arzneimittelbehälter umfasst, der innerhalb der Nadelabdeckung und des Gehäuses eingerichtet ist; und
eine Kappenbaugruppe (30), die konfiguriert ist, um an dem Gehäuse montiert zu werden, um eine proximale Öffnung des Gehäuses abzudecken, wobei die Kappenbaugruppe umfasst
ein Verriegelungselement (60), einschließlich eines ersten Eingriffselements (61), das konfiguriert ist, um mit der Nadelabdeckung und dem Gehäuse zu interagieren, um eine Bewegung der Nadelabdeckung zu einer Innenseite des Gehäuses hin einzuschränken, wobei das erste Eingriffselement radial flexibel ist und zwischen dem Gehäuse und der Nadelabdeckung eingerichtet ist;
und eine Kappe (40), die konfiguriert ist, um das Verriegelungselement in Eingriff zu nehmen, und die in Bezug auf das Gehäuse und das Verriegelungselement zwischen einer ersten Position, in der die Kappe das Gehäuse in Eingriff nimmt, und einer zweiten Position, in der die Kappe das Gehäuse außer Eingriff bringt, bewegbar angeordnet ist;
wobei die Nadelabdeckung ein zweites Eingriffselement (71) einschließt, das konfiguriert ist, um das erste Eingriffselement in Eingriff zu nehmen, wenn sich die Kappe zwischen der ersten Position und der zweiten Position befindet;
wobei das zweite Eingriffselement eine Nut ist, die an der Außenoberfläche der Nadelabdeckung ausgebildet ist, und wobei das Verriegelungselement ferner einen Anschlagnocken (62) einschließt, der an dem Ende des ersten Eingriffselements eingerichtet ist, der konfiguriert ist, um in der Nut der Nadelabdeckung zu ruhen;
wobei die Kappe ferner bewegbar zwischen der zweiten Position und einer dritten Position angeordnet ist, in der der Anschlagnocken des Verriegelungselements die Nut der Nadelabdeckung außer Eingriff bringt und die Bewegung der Nadelabdeckung nicht länger einschränkt, wobei die Kappe ein drittes Eingriffselement einschließt und das Verriegelungselement ein viertes Eingriffselement einschließt, das dritte Eingriffselement konfiguriert ist, um das vierte Eingriffselement in Eingriff zu nehmen, wenn sich die Kappe von der ersten Position in die zweite Position bewegt, und das dritte Eingriffselement konfiguriert ist, um das Verriegelungselement von dem Gehäuse weg zu bewegen, wenn sich die Kappe von der zweiten Position in die dritte Position bewegt.

2. Arzneimittelabgabevorrichtung nach Anspruch 1,
**dadurch gekennzeichnet, dass** ein Ende des Gehäuses konfiguriert ist, um das erste Eingriffselement in Eingriff zu nehmen, um zu verhindern, dass sich das erste Eingriffselement von der Nadelabdeckung weg bewegt, wenn sich die Kappe zwischen der ersten und zweiten Position befindet.

3. Arzneimittelabgabevorrichtung nach Anspruch 1,
**dadurch gekennzeichnet, dass** die Kappenbaugruppe ferner einen Nadelschutzentferner (50), der konfiguriert ist, um das Verriegelungselement in Eingriff zu nehmen, und einen Nadelschutz (84) umfasst, der an einem Arzneimittelbehälter innerhalb der Nadelabdeckung und des Gehäuses angebracht ist, wobei, wenn sich die Kappe von der zweiten Position in die dritte Position bewegt, die Kappe das Verriegelungselement bewegt, um den Nadelschutzentferner von dem Arzneimittelbehälter weg zu bewegen, um den Nadelschutz von dem Arzneimittelbehälter weg zu ziehen.

4. Arzneimittelabgabevorrichtung nach Anspruch 1,
**dadurch gekennzeichnet, dass** die Kappe eine Vielzahl von Greifvorsprüngen (41) an einer Außenoberfläche der Kappe einschließt, die konfiguriert sind, um mit einem Benutzer zu interagieren, wenn der Benutzer die Kappe greift.

5. Arzneimittelabgabevorrichtung nach Anspruch 1,
**dadurch gekennzeichnet, dass** das Gehäuse ein fünftes Eingriffselement (21) an einer Außenoberfläche des Gehäuses einschließt, wobei die Kappe mindestens ein sechstes Eingriffselement (42) an einer Innenoberfläche der Kappe einschließt, wobei das sechste Eingriffselement konfiguriert ist, um das fünfte Eingriffselement in Eingriff zu nehmen, damit die Kappe an dem Gehäuse montiert werden kann.

## Revendications

1. Dispositif d'administration de médicament (10) comprenant :
un logement (20) ;
un couvre-aiguille (70) placé de manière déplaçable au moins partiellement dans le logement :
dans lequel le dispositif d'administration de médicament comprend en outre un récipient de médicament disposé au sein de la protection d'aiguille et du logement ; et
un ensemble capuchon (30) conçu pour être monté au logement pour couvrir une ouverture proximale du logement, dans lequel l'ensemble capuchon comprend
un élément de verrouillage (60) comportant un premier élément de mise en prise (61) conçu pour interagir
avec le couvre-aiguille et le logement pour limiter un déplacement du couvre-aiguille en direction d'un intérieur du logement, dans lequel le premier élément de mise en prise est radialement flexible et est disposé entre le logement et le couvre-aiguille ; et un capuchon (40) conçu pour venir en prise avec l'élément de verrouillage et agencé de manière mobile par rapport
au logement et à l'élément de verrouillage entre une première position dans laquelle le capuchon est en prise avec le logement et une deuxième position dans laquelle le capuchon se désolidarise du logement ;
dans lequel le couvre-aiguille comporte un deuxième élément de mise en prise (71) conçu pour venir en prise avec le premier élément de mise en prise lorsque le capuchon est entre la première position et la deuxième position ;
dans lequel le deuxième élément de mise en prise est une rainure formée sur la surface externe du couvre-aiguille, et dans lequel l'élément de verrouillage comporte en outre une came d'arrêt (62) disposée au niveau de l'extrémité du premier élément de mise en prise conçue pour reposer au sein de la rainure du couvre-aiguille ;
dans lequel le capuchon est en outre agencé de manière mobile entre la deuxième position et une troisième position dans laquelle la came d'arrêt de l'élément de verrouillage désolidarise la rainure du couvre-aiguille et ne limite plus le déplacement du couvre-aiguille dans lequel le capuchon comporte un troisième élément de mise en prise et l'élément de verrouillage comporte un quatrième élément de mise en prise, le troisième élément de mise en prise est conçu pour venir en prise avec le quatrième élément de mise en prise lorsque le capuchon se déplace de la première position à la deuxième position, et le troisième élément de mise en prise est conçu pour déplacer l'élément de verrouillage à l'écart du logement lorsque le capuchon se déplace de la deuxième position à la troisième position.

2. Dispositif d'administration de médicament selon la revendication 1,
**caractérisé en ce qu'**une extrémité du logement est conçue pour venir en prise avec le premier élément de mise en prise pour empêcher le premier élément de mise en prise de s'éloigner du couvre-aiguille lorsque le capuchon est localisé entre les première et deuxième positions.

3. Dispositif d'administration de médicament selon la revendication 1,
**caractérisé en ce que** l'ensemble capuchon comprend en outre un dispositif de retrait de protection d'aiguille (50) conçu pour venir en prise avec l'élément de verrouillage et une protection d'aiguille (84) fixée à un récipient de médicament au sein du couvre-aiguille et le logement, lorsque le capuchon est déplacé de la deuxième position à la troisième position le capuchon déplace l'élément de verrouillage pour déplacer le dispositif de retrait de protection d'aiguille à l'écart du récipient de médicament pour tirer la protection d'aiguille à l'écart du récipient de médicament.

4. Dispositif d'administration de médicament selon la revendication 1,
**caractérisé en ce que** le capuchon comporte une pluralité de parties saillantes de préhension (41) sur une surface externe du capuchon conçues pour interagir avec un utilisateur lorsque l'utilisateur saisit le capuchon.

5. Dispositif d'administration de médicament selon la revendication 1,
**caractérisé en ce que** le logement comporte un cinquième élément de mise en prise (21) sur une surface externe du logement, le capuchon comporte au moins un sixième élément de mise en prise (42) sur une surface interne du capuchon, le sixième élément de mise en prise est conçu pour venir en prise avec le cinquième élément de mise en prise afin que le capuchon soit monté sur le logement.
